# EUROPEAN PATENT SPECIFICATION

(11) **EP 2 696 900 B1**
(45) Date of publication and mention of the grant of the patent: **17.01.2018**
(21) Application number: 12765341.8
(22) Date of filing: 29.03.2012
(51) Int. Cl.: A61K 49/00, G01N 1/30

(54) **DYES FOR MEMBRANES AND BIOLOGICAL STRUCTURES**
FARBSTOFFE FÜR MEMBRANEN UND BIOLOGISCHE STRUKTUREN
COLORANTS POUR MEMBRANES ET STRUCTURES BIOLOGIQUES

(30) Priority: 29.03.2011 US 201161468838 P; 29.03.2012 US 201213433526
(43) Date of publication of application: 19.02.2014
(73) Proprietor: Kemin Industries, Inc., Des Moines, Iowa 50317 (US)
(72) Inventor: DE SOUSA MARTINS, Diogo, 01404-000 Sao Paulo (BR); RUBENS, JR., Belfort, 01413-100 Sao Paulo (BR); DE SOUZA FILHO, Acacio Alves, 01402-002 Sao Paulo (BR); MAIA, Mauricio, 19060-010 Presidente Prudente (BR)
(74) Representative: Evans, Jacqueline Gail Victoria
(86) International application number: PCT/US2012/031098
(87) International publication number: WO 2012/135432

(56) References cited:
- WO-A1-2010/125516
- US-A- 5 596 011
- US-A1- 2004 254 567
- US-A1- 2008 254 140
- US-A1- 2009 035 369
- US-A1- 2011 021 465
- US-A1- 2011 052 678
- WU ET AL.: 'Phototoxicity of Indocyanine Green on Human Retinal Pigment Epithelium in Vitro and its Reduction by Lutein.' PHOTOCHEMISTRY AND PHOTOBIOLOGY vol. 81, no. 3, May 2005, pages 537 - 540, XP055137839

## Description

The present invention relates to the use of one or more natural dyes, alone or in combination with other dyes, for preparation of a composition to stain ocular membranes and structures in order to facilitate their identification during intraocular surgical procedures and to the use of one or more natural dyes, alone or in combination with other dyes, to stain ocular membranes and structures in order to facilitate their identification during intraocular surgical procedures and their compositions. The present invention also relates to one or more natural dye compositions for use in protecting tissues and cells exposed during surgical procedures from damage by light.

### Background of the Invention

Surgical procedures, in various fields of medicine, often require the visualization and, in some cases, the removal of delicate membranes, which in most cases are thin and semitransparent. During some surgical procedures, it is necessary to remove these membranes. This step is technically very complex, given the difficulty of identifying each membrane adequately.

The intraoperative utilization of vital dyes for staining of human tissues has been established for more than three decades. The use of vital dyes in ophthalmology to stain retinal tissues dates back to the last century.³⁵ Beginning with Sorsby in 1939³⁶ and later Gifford in 1940,³⁷ ophthalmologists began injecting Kiton-fast-green V, Xylene-Fast-green B, and fluorescein intravenously. In 1969, Kutschera began injecting patent blue intravitreally to stain retinal tissue and determine retinal breaks in retinal detachments.³⁸

The introduction of vitreoretinal surgery, pars plana vitrectomy, allowed the treatment of serious diseases of the retina such as diabetic retinopathy, macular hole and retinal detachment. The vitrectomy surgical technique involves removing the intraocular vitreous gel, taking off all pre-retinal membranes, and restoring the ocular volume with a balanced salt solution, gas or silicone oil. The removal of pre-retinal membranes such as in the treatment of proliferative vitreoretinopathy, as a consequence of retinal detachment, fibroglials epiretinal membranes and internal limiting membrane (ILM), consists of a technically difficult surgical step due to the thin and semi-transparent nature of the tissues.^{1,2} Several studies have shown retinal complications caused iatrogenically by the action of removing these micro-structures.

The use of intraoperative dyes in chromovitrectomy is one of the most recent advances in vitreoretinal surgery³. This technique was first described by Burk et al. in 2000. In this work the vital dye indocyanine green (ICG) showed high affinity for fine ILM⁴. The previously difficult task of removing the ILM and epiretinal membranes in vitreoretinal surgery was made much easier, increasing the rates of anatomic and functional success⁵⁻⁶. However, the initial enthusiasm was replaced by concern after clinical trials demonstrated clinical postoperative complications related to use of ICG such as visual field and retinal pigment epithelium changes⁷⁻¹². Studies in vitro and in vivo in different animal models such as mice, rabbits and pigs, have determined dose-dependent ICG toxicity in retinal cells¹³⁻¹⁶. The harmful effect of ICG during chromovitrectomy motivated the scientific community to research vital dyes that are less toxic. Some of the first dyes used in research and clinical use in chromovitrectomy are trypan blue (AT) and patent blue (AP). However the acceptance of these dyes has been limited because both have low chemical affinity for ILM and other pre-retinal membranes, and there are no precise indicators of their biosecurity¹⁷⁻²¹.

Since then, several researchers around the world have proposed to study this further in order to find a dye with high capacity and staining of the intraocular structures that is safe for the retina. In the search for the ideal dye, the present invention proposes the use of natural dyes, including the use of a pigment that is a constitutive structure of the retina, as a new alternative dye for use in chromovitrectomy or other surgeries or procedures where it is necessary or helpful to define layers or boundaries to guide a health care provider during a procedure or process.

The document PI0311609-3 reveals compositions containing lutein and zeaxanthin and the use of such compositions as a source of nutrition for infants, whose purpose, as well as the composition, dosage, formulation, mode of administration, use context and production process are completely distinct to those revealed in the present invention.

The document JP2008138158 refers to a yellow pigment extracted from cocoons cricula, and a yellow dye, an artificial color, a UV-filtering agent and an antioxidant.

The document EP1075284B1 refers to the use of vital dyes to facilitate surgical procedures in vitreoretinal surgery.

US 2011/052678 and WO 2010/125516 disclose compositions comprising lutein and zeaxanthine for the topical treatment of eye diseases such as macular degeneration.

The use of indocyanine green in dye-assisted peeling of the internal limiting membrane during vitroretinal surgery is described in Photochem. Photobiol 81(3), 537 (2005).

The present invention is distinguished by the fact that the vital dyes used in this invention are natural dyes, alone or in combination with other dyes, while the dyes mentioned in the document above are all synthetic.

The present invention also provides one or more natural compositions that can be used to protect tissues and cells exposed during surgical procedures from damage due to light present in the surgical field. The compositions include lutein and zeaxanthin which absorb light and provide a protective effect, particularly against the most damaging wavelengths of light.

The compositions can be used to protect intraocular tissues and cells by topical application to the eye or injection into the eye.

### Summary of the Invention

The present invention relates to the use of one or more natural dyes, alone or in combination with other dyes, to stain ocular membranes and structures in intraocular surgery and the use of such compounds for the preparation of compositions to stain ocular membranes and structures with the purpose of facilitating the identification of these during intraocular surgical procedures.

The present invention further relates to compositions comprising one or more natural dyes, alone or in combination with other dyes, in combination with pharmaceutically acceptable vehicles and structures, to identify ocular membranes during intraocular surgical procedures.

An object of the present invention is the protection of tissues and cells exposed during surgical procedures from damage due to light present in the surgical field by the administration of one or more dyes that incorporate lutein and zeaxanthin, which absorb light.

Intraocular tissues and cells may be protected by topical application to the eye (externally or internally) or injection into the eye (externally or internally) of one or more dyes that include natural substances such as lutein and zeaxanthin.

### Brief Description of Figures

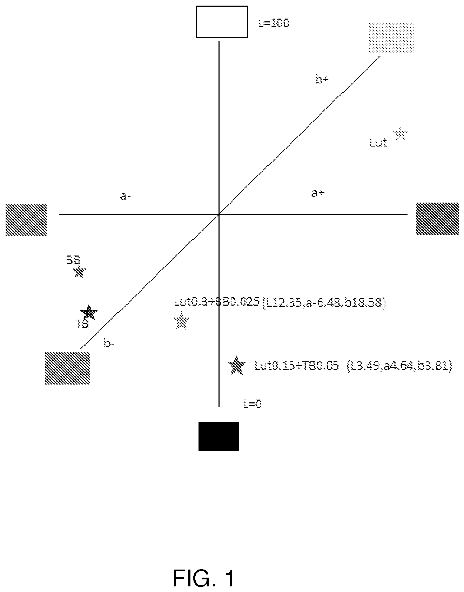

Fig. 1 is a colorimetry chart showing the locations of various dyes and combinations of dyes of the present invention.

### Detailed Description of the Invention

The present invention relates to compositions comprising one or more natural dyes, alone or in combination with other dyes, in combination with pharmaceutically acceptable vehicles, for use in the identification of intraocular membranes and structures during intraocular surgical procedures.

The ocular membrane or structure is selectively stained such that biological tissue adjacent the selected membranes or structures are not stained to a substantial extent, or at least to a significantly lesser, non-detrimental extent than the membranes or structures selected to be stained. Accordingly, during the surgical procedure, the selected biological membrane or structure is clearly distinguishable from adjacent tissues and so facilitates the surgical procedure and reduces the risk of damage to adjacent tissues.

Using dyes of the present invention, little or no detrimental effect on adjacent tissues has been observed. Further, any remaining dye in the surgical field is removed shortly after the procedure, thereby reducing the possibility of adverse side effects of the dye.

Dyes of the present invention create a satisfactory level of staining at a concentration which is physiologically and toxicologically acceptable. It is preferred that a minimum amount of dye be used that will still provide a visible differentiation between the selected ocular membranes or structures and the adjacent tissue.

The dyes of the present invention are selected from the group comprising ingredients, portions or extracts of lutein, zeaxanthin, urucum, lycopene, astaxanthin, beta-carotene, and beta-cryptoxanthin, alone or in combination with each other. Such dyes may also be used in combination with dyes selected from the group comprising ingredients, portions or extracts of brilliant blue, indocyanine green, infracyanine green, trypan blue, patent blue, bromophenol blue, fast green, indigo carmine, evans blue, light green, triamcinolone, crystal violet, fluorescein, fluormetolone acetate and congo red. In a more particular embodiment, the compositions comprise lutein and zeaxanthin alone or combined with trypan or brilliant blue.

The pharmaceutically acceptable vehicles can be selected from the group comprising phosphate buffer, balanced salt solution (BSS), polyvinyl alcohol, benzyl alcohol, purified water, sodium phosphate, sodium chloride, calcium chloride, magnesium chloride, potassium chloride, sodium citrate , sodium acetate, boric acid, borax, methylcellulose and derivatives, hyaluronic acid, dextran sodium polysorbate, tweens, chondroitin sulfate, sodium edetate, propylene glycol, polyethylene glycol, phenylphosphate, sodium phthalate, potassium phosphate, citric acid, carbomer 934, carbopol, metaphosphoric acid, glycocholic acid and acetic acid. The expressions Carbopol, Tween and Carbomer 934 are registered trade marks.

The compositions of the present invention may comprise from 0.001% to 20%, preferably 0.01 % to 5%, and including all ratios in between these recited limits, of the dye.

The intraocular membranes referred to in this invention are preferably selected from the group comprising the anterior and posterior capsule, corneal epithelium and endothelium, epiretinal membrane, internal limiting membrane, vitreous, posterior hyaloid and other ocular membranes.

The invention is
applicable in the identification of intraocular membranes and biological structures in ophthalmic surgeries including chromovitrectomy. Preferably, the dyes used in this invention are lutein and zeaxanthin and their combinations.

Lutein and zeaxanthin, besides being carotenoids, and more particularly xanthophylls, are known and studied for their association with the prevention of age-related maculopathy. The present invention relates to the use of lutein and/or zeaxanthin as safe and effective dyes or stains for surgical procedures, such as, among others, chromovitrectomy, allowing an easy identification of membrane and biological structures such as epiretinal membrane, ILM, anterior and posterior capsule, corneal epithelium and endothelium , vitreous and posterior hyaloid, and their complete removal, when necessary, by the ophthalmic surgeon.

In view of the iatrogenic retinal complications caused by the procedures in removing these micro-structures shown in prior art, the use of natural colorants, alone or in combination with other dyes, to stain intraocular membranes and other structures, enable a breakthrough both in the surgical technique as well as in the toxicity profile, with substantial improvements in surgical outcomes.

Lutein, which has the structural formula below: 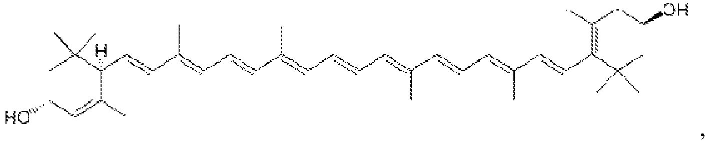 and zeaxanthin, which has the structural form below: 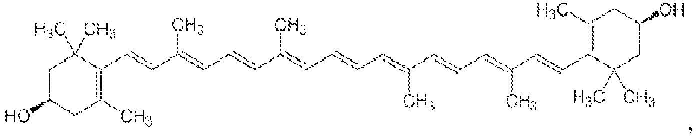 are lipophilic pigments belonging to the group of carotenoids and traditionally found in fruits and vegetables. These two carotenoids are structural isomers and have a hydroxyl group in the terminal portion of the molecule, which partly explains their different polarity and tropism for certain biological structures, such as the macula. Lutein and zeaxanthin are associated with the possible prevention of age-related maculopathies due both to their antioxidant properties, which can prevent progress of macular degeneration; and to their recognized distribution in the macula. This selective and specific distribution of these carotenoids suggests that they play an important role in this region, both as a "filter" of blue light, which typically induces the formation of radical oxygen species, as well as their activity as antioxidants. The role of lutein and zeaxanthin in the eye is a good example of how the human body can defend itself against an oxidative process. Interestingly, lutein and zeaxanthin have a dual and synergistic mechanism of action in the macula. Lutein and zeaxanthin are present in the central yellow region (the fovea) having a maximum absorption at 446 nm, which is coincidentally the same wavelength as blue light in the electromagnetic spectrum, thus, absorbing the excess photon energy. Exactly because of this absorption, lutein and zeaxanthin have chromophore groups responsible for the their characteristic yellow-orange color.

In Europe, lutein is classified as a food dye, registered under number E161b in EFSA (European Food Safety Authority). The fact that it is (i) a GRAS molecule (Generally Recognized as Safe, the highest distinction awarded to a food / additive / dye), (ii) recognized by the FDA (Food & Drug Administration in the United States of America) as such, (iii) widely studied and the object of several scientific papers published in peer-reviewed journals, (iv) associated with a possible beneficial effect in preventing today's major retinal diseases and (v) a dye, makes it an interesting substance as an object of study and identification of intraocular membranes and other biological structures, not limited to, for example, intraocular membranes.

In Europe, zeaxanthin is also registered as a food dye by EFSA and has been assigned E number E161h. Zeaxanthin is a GRAS molecule and has been the subject of scientific papers published in peer reviewed journals. As dye, zeaxanthin makes an interesting substance as an object of study and identification of intraocular membranes biological structures, not limited to, for example, intraocular membranes.

The combined use of lutein and and/or zeaxanthin, alone or in combination with other substances such as dyes and other pharmaceutically acceptable vehicles, as a dye composition of the anterior and posterior capsule, corneal epithelium and endothelium, epiretinal membrane, internal limiting membrane, the vitreous and posterior hyaloid, is illustrated along with data pertaining to its actions through examples described below.

Despite the fact that certain definitions about lutein and zeaxanthin dyes and their combinations in the field of ophthalmic surgery are described in more detail here, the invention may also be achieved with other dyes and application fields previously defined.

Table 1 below lists dyes that can be used in the invention and their particular quantitative ranges.

**Table 1**

| Component | Concentration [% w/v] | Preferable Concentration [% w/v] | Function within the composition |
|---|---|---|---|
| Lutein | 0.001% to 20% | 0.01% to 5% | Active |
| Zeaxanthin | 0.0007% to 20% | 0.007% to 0.5% | Active |
| Urucum | 0.01% to 20% | | Active |
| Brilliant Blue | 0.01% to 0.05% | 0.0125% to 0.025% | Active |
| Green Indocianine | 0.001% to 20% | 0.5% | Active |
| Green Infracianine | 0.001% to 20% | 0.5% | Active |
| Trypan Blue | 0.001% to 20% | 0.04% | Active |
| Patent Blue | 0.05% to 0.5% | 0.24% | Active |
| Bromophenol Blue | 0.05% to 0.5% | 0.2% | Active |
| Fast green | 0.001% to 20% | | Active |
| Indigo Carmine | 0.001% to 20% | | Active |
| Evans Blue | 0.001% to 20% | | Active |
| Light Green | 0.001% to 20% | | Active |
| Triancinolone | 0.001% to 20% | | Active |
| Crystal Violet | 0.001% to 20% | | Active |
| Fluorescein | 0.001% to 20% | | Active |
| Fluormetolone Acetate | 0.001% to 20% | | Active |
| Congo Red | 0.001% to 20% | | Active |
| Phosphate Buffer | 0.001% to 99.99% | | Vehicle |
| Balanced Saline Solution | 0.001% to 99.99% | | Vehicle |
| Polyvynilic Acid | 0.001% to 99.99% | | Vehicle |
| Benzylic Acid | 0.001% to 99.99% | | Vehicle |
| Purified Water | 0.001% to 99.99% | | Vehicle |
| Sodium Phosphate | 0.001% to 99.99% | | Vehicle |
| Sodium Cloride | 0.001% to 99.99% | | Vehicle |
| Calcium Cloride | 0.001% to 99.99% | | Vehicle |
| Magnesium Cloride | 0.001% to 99.99% | | Vehicle |
| Potassium Cloride | 0.001% to 99.99% | | Vehicle |
| Sodium Citrate | 0.001% to 99.99% | | Vehicle |
| Sodium Acetate | 0.001% to 99.99% | | Vehicle |
| Boric Acid | 0.001% to 99.99% | | Vehicle |
| Sodium Borate | 0.001% to 99.99% | | Vehicle |
| Methylcelulose and its derivatives | 0.001% to 99.99% | | Vehicle |
| Hyaluronic Acid | 0.001% to 99.99% | | Vehicle |
| Dextrane | 0.001% to 99.99% | | Vehicle |
| Sodium Polisorbate | 0.001% to 99.99% | | Vehicle |
| Tweens | 0.001% to 99.99% | | Vehicle |
| Chondroitin Sulfate | 0.001% to 99.99% | | Vehicle |
| Sodium Edetate | 0.001% to 99.99% | | Vehicle |
| Propyleno glycol | 0.001% to 99.99% | | Vehicle |
| Polyethyleno glycol | 0.001% to 99.99% | | Vehicle |
| Sodium Phenylphosphate | 0.001% to 99.99% | | Vehicle |
| Potassium Phthalate | 0.001% to 99.99% | | Vehicle |
| Potassium Phosphate | 0.001% to 99.99% | | Vehicle |
| Citric Acid | 0.001% to 99.99% | | Vehicle |
| Carbomer 934 | 0.001% to 99.99% | | Vehicle |
| Carbopol | 0.001% to 99.99% | | Vehicle |
| Metaphosforic Acid | 0.001% to 99.99% | | Vehicle |
| Glycocol | 0.001% to 99.99% | | Vehicle |
| Acetic Acid | 0.001% to 99.99% | | Vehicle |

There are many benefits associated with the use of natural dyes, including: (i) easy identification of membrane and biological structures to identify, remove, or work with; (ii) prevention of side effects caused by incomplete removal of membranes and biological structures; (iii) reducing the time of surgery with reduction in medical and hospital fees; (iv) increased safety because the proposed dye is a natural dye, alone or in combination with other dyes, not synthetic, and all the alternatives on the market today have demonstrated toxicity; and (v) possible antioxidant effect associated with the use of dye in the surgery or other medical procedures.

A general process of the preparation of compositions of the present invention is as follows. The packaging material is received. Subsequently, the raw materials needed for manufacturing are properly weighed. The handling and / or filtration is done in ISO 8 class room. Before septic or aseptic filling, in-process quality control is performed. If the filling is aseptic, it follows the following flow: Filling in the ISO 5 class room, Sealing in ISO 7 class room and Sterilization in the final sterilization room. Conversely, if the filling is septic, it follows the following flow: Sterilizing Filtration in ISO 5 class room, Filling in ISO 5 class room and Sealing in ISO 7 class room. After these steps, packaging and quality control of the finished product takes place.

The manufacturing process of compositions comprising one or more dyes or stains, such as lutein, zeaxanthin, urucum, lycopene, astaxanthin, beta-carotene, beta-cryptoxanthin, alone or in combination with each other, and such dyes and dye compositions alone or in combination with brilliant blue, indocyanine green, infracyanine green, trypan blue, patent blue, bromophenol blue, fast green, indigo carmine, Evans blue, light green, triamcinolone, crystal violet, fluorescein, fluormetolone acetate and congo red. Those skilled in the art will be able to utilize mixtures of these dyes as long as the mixture accomplishes the purposes of staining selected biological tissues to readily distinguish them from adjacent biological tissues, preferably at low amounts and with no, or very little, toxicity, and with no, or very little, dye remaining after the procedure. In general, the concentration of the dye or mixture of dyes is minimized while still effectively staining the selected biological tissue while at the same time minimizing the risk of possible damage or toxicity to the biological tissue. The viscosity of the dye solution may be adjusted, for example by the addition of hyaluronic acid, to achieve improved adherence to the selected biological tissue. In addition, the solution may be formulated as a dispersion. It is well within the skill in the art to select and formulate a suitable form of the solution for particular applications.

### Example 1 - Staining of Human Eye Tissue with Lutein and/or Zeaxanthin

Forty human eyes were tested using solutions of other substances including lutein/zeaxanthin(L/Z) (Lutein 5% CWS/S-TG from DSM Nutritional Products, Inc., Parsippany, NJ), brilliant blue (OPHT-Blue from Ophthalmos Industry, Sao Paolo) and / or other vehicles at concentrations of lutein from 0.01% to 20% and brilliant blue from 0.0125% to 0.025%. The staining solutions were prepared as described in Example 2. The anterior capsule, vitreous, posterior hyaloid and corneal endothelium were stained through immediate deposition of the dye solution in the membrane.

The following scoring technique was used for the cornea:

| **Score** | **Corneal Grading Criteria**¹ |
|---|---|
| 0 | No Stain Retention. |
| 1 | One Quarter (or less) but not zero. |
| 2 | Greater than one quarter, but less that one half. |
| 3 | Greater than one half, but less than three quarters. |
| 4 | Greater than three quarters up to the whole area. |

| | |
|---|---|
| ¹ Draize, J.H., et al., J. Pharm. Exp. Ther., 82:377-390, 1944. | |

For the ILM the scoring used was: 0/4+ (none), 1+/4+ (weak), 2+/4+ (regular), 3+/4+ (good), 4+/4+ (intense).

Staining of the anterior capsule was observed of 3 + / 4 + and 4 + / 4 +, respectively with a solution of L/Z 0.25% and brilliant blue 0.025% and a solution of L/Z 0.5% and brilliant blue 0.025%. This staining degree received a score of 1+/4+ with a solution of L/Z at 1%. It was also observed the staining of the vitreous at 4+/4+ with a solution of L/Z at 20% as well as staining the ILM at 4+/4+ with a solution of L/Z at 0.3% and 0.025 brilliant blue. The solution of L/Z at 1% alone also stained the ILM at 2+/4+. It was observed a staining of corneal endothelium at 2+/4+ with a solution of L/Z at 20%.

These experiments establish that lutein and zeaxanthin in combination with vehicles and/or pharmaceutically acceptable dyes, can be used for the preparation of a composition to facilitate the identification of membranes and biological structures during ophthalmic surgical procedures.

The quantities of dyes in general, and more specifically, lutein and zeaxanthin, as well as vehicles and/or pharmaceutically acceptable dyes are listed in Table 1.

While either lutein or zeaxanthin may be used alone or in combination with other dyes, the preferred proportion of lutein to zeaxanthin in the composition ranges from 5:1 to 1:5 and all values in between.

### Example 2 - Composition of lutein and/or zeaxanthin and brilliant blue:

The preparation technique described below is valid for L/Z, whatever its chemical form, purity, crystallization or degree of esterification and for all the vehicles mentioned above.

L/Z does not exhibit good water solubility, therefore, polyvinyl alcohol was used as a vehicle at a concentration of 1.4% dissolved in phosphate buffer at pH 7.0. This alcohol can be dissolved in another buffer or a simple or balanced saline solution.

### Materials:

L/Z 0.5 g
Brilliant blue: 0.025 g
Monobasic sodium phosphate: 0.004 g
Dibasic sodium phosphate. 0.028 g
Sodium chloride. 0.85 g
Povidone 0.4 g
Polyvinyl Alcohol 1.4 g
Water q.s.p. 100 mL

### Preparation:

Weigh the active component: Brilliant Blue and L/Z and, under stirring, dissolve it in buffer solution (pH: 7.0 to 7.4) containing 1.4% polyvinyl alcohol.

Check the pH.

Proceed with filling in clean area ISO Class 5 (Class 100) into a vial with a rubber stopper and a Flip-Off aluminum seal containing 0.5 mL. (Single dose).

Sterilize by autoclaving for 30 min at 121 °C.

The process starts by dissolving the components in a balanced salt solution, or any other solution that has compatibility with the components of the formula and to provide stability, pH and osmolality compatible with ocular use, including various forms and dosages of the active components and their vehicles. Some examples of formulas are included in Table 2.

**Table 2 - Exemplary Composition Formulations**

| **#** | **Color** | **% w/v of a first or only dye** | **% w/v of a second dye** |
|---|---|---|---|
| 1 | Greenish Blue | 0.50% L/Z | 0.05% Brilliant Blue |
| 2 | Greenish | 0.50% L/Z | 0.025% Brilliant Blue |
| 3 | Green | 0.50% L/Z | 0.0125% Brilliant Blue |
| 4 | Greenish Blue | 0.25% L/Z | 0.025% Brilliant Blue |
| 5 | Greenish | 0.25% L/Z | 0.0125% Brilliant Blue |

Other embodiments prepared include: (1) 20% L/Z dispersible / soluble in water regardless of physical form and polyvinyl acid 1.4% qs 100%; (2) 0.5% L/Z dispersible / soluble in water regardless of physical form, 0.025% brilliant blue, and polyvinyl acid 1.4% qs 100%; and (3) 0.5% L/Z dispersible / soluble in water regardless of physical form, 0.0125% brilliant blue, and polyvinyl acid 1.4% qs 100%.

The main manipulation techniques used include: (i) dissolution; (2) agitation; (3) filtration; and (4) sterilization.

The sterilization step, when necessary, is either performed through wet heat, by autoclaving with temperatures ranging from 80° to 140°C, by sterilizing filtration, by radiation or by other compatible method.

The pH is observed in a range from 3 to 12 and osmolarity and osmolality ranges from 100 to 2000 mOsm, with preferred formulations having osmolality around 300mOsm.

### Example 3 - Development of Intraocular Formulations

Besides the use of one dye alone in a composition, as previously mentioned, it is also possible to prepare a composition comprising of a combination of more than one dye. In this invention, not only are vehicle and active concentrations supplied and identified that differ from prior art, but also the specific use and site of deposition, which is made directly into the eye membranes or other biological structures. This was not provided by prior art where lutein and zeaxanthin were administered orally (capsules, tablets, cereal bars, or fortified milks). The present invention provides a composition in the form of an injectable dye formulated from the raw material including lutein and/or zeaxanthin. The prior art did not teach compositions that could be administered during surgical procedures in an injectable administration form. Specific applications include an injection solution or dispersion, an ocular implant for protection against macular degeneration or the progression of macular degeneration, and a slow-release form of L/Z placed in the eye.

### Lutein and/or Zeaxanthin

The dissolution is done with the vehicles identified in Table 1 above. The solution of L/Z is weighed and diluted with distilled water in various concentrations ranging between 0.01% and 20%. The osmolality of the solutions is calculated by the ATM Osmomette osmometer (Precision Systems, Inc., Sudbury, MA) or other applicable osmometers. A balanced salt solution (BSS) is used as the control using an osmolarity or osmolality from 100 to 2000 mOsm.

The main steps are listed below:
1. To determine a vehicle from Table 1 so that the final solution is water soluble, solubility tests in water and in various vehicles including those shown in Table 1 were performed. Solubilities ranging between 0.02 and 110 mg / m were found.
2. To characterize the absorbance of the L/Z solution, UV spectrophotometry was used. Absorbency between 2-5 within a wavelength range of 250nm to 1000nm at a speed of 600nm/min and using 1.4% polyvinyl alcohol as a white standard were observed.
3. To identify the resulting color of the chromophore groups, mixtures comprising different colors within the visible spectrum were analyzed.
4. To test the physicochemical parameters of optimum pH, osmolarity/osmolality, and concentration, the procedures described above were followed.

### Example 4 - Cadaveric Eyes Study

One hundred and twenty human cadaver eyes from human corneal donors were tested using solutions lutein and zeaxanthin alone or combined with brilliant and trypan blue.. Initially, the anterior part of the eye or the cornea was removed through limbal incision 360 degrees as routine, and kept for a cornea transplant. The rest of the eyeball was usually discarded, with the only exception of the scleral tissue that can eventually be used as an implant to correct human scleral thinning or in enucleation surgery. Before the elimination of most ocular tissues and sclera preparation, experiments were performed in coloring different eye membranes and intraocular structures.

Soon after the removal of the cornea, 0.1-0.3 mL of the dye (L/Z 0,25-20% alone or combined with brilliant blue (0.0125% -0.025%) or Trypan Blue (0.01% - 0.15%) were applied to the anterior capsule of the lens. After 30 seconds the dye was removed with a light irrigation of 10 mL of BSS. Then the capsule of the lens was removed with Utrata capsulorhexis forceps by applying circular motion with 360 deg. After removal of excess dye, the staining intensity was measured by grade 0 (no staining), + (slight staining), + + (moderate staining), + + +/++++ (intense staining), by three experienced surgeons who did not know the substances and who were operating independently. The anterior capsules removed were evaluated by spectrophotometry.

The lens was removed for the experiments by using a surgical forceps for vitreous excision in block. The entire vitreous was immersed in 1 mL of each dye solution for 1 minute, followed by the immersion of the same vitreous in 1 mL of BSS.

In the last experiment, the binding capacity of the dye in the ILM was evaluated after intravitreal injection of the ILM. A total of 0.1-0.3 mL of each dye solutionwas injected into the vitreous cavity on the ILM. The excess dye was removed by an infusion of 0.1 mL of BSS. After removal of excess dye, the intensity of staining in the ILM and vitreous were evaluated grade 0 (no staining), + (slight staining), + + (moderate staining), + + +/++++ (intense staining) by three vitreoretinal surgeons experts who do not know the substances used and who were operating independently. Then a bent 27-gauge needle was used to create a flap on the ILM, and a 20-gauge vitrectomy surgery pinch is used to hold and gently remove the ILM. The ILMs removed were evaluated by means of spectrophotometry.

### Example 5 - Histological analysis

ILM and anterior capsule of the lens were removed and fixed in a solution of formalin at 10% for 24 hours. This was followed by soaking the tissue in Araldite® (Huntsman International, LLC) for histological sections. The material was collected from two different areas at sites of the previous injections of each dye solution. All samples were collected in areas separated by a minimum of 500 µm from each other. The samples were analyzed by light microscopy and electron microscopy. For light microscopy, the thin tissue sections were examined after staining with toluidine blue dye. For electron microscopy ultrathin sections were placed on copper micro-supports or grids, stained with uranyl acetate and silver citrate, and examined with electron microscope Jeol JX 1500TM (Jeol Ltd., Tokyo, Japan).

### Example 6 - Toxicity Study in Rabbits

Thirty Dutch-belted rabbits weighing 1.5 to 2 kg were used according to the research standards of the Helsinki Declaration, and the Rules of the Association for Research in Vision and Ophthalmology (ARVO) and the Standards of Ethics in Research of the University of Sao Paulo regarding experiments on animals. All surgical procedures and tests were done under anesthesia after intramuscular injection of 35mg/kg ketamine hydrochloride (Phoenix Scientific Inc., USA) and 5 mg/kg of ketamine hydrochloride (Phoenix Scientific Inc., USA). The pupil was dilated before surgery through the instillation of cyclopentolate hydrochloride 1% (Bausch & Lomb Pharmaceuticals Inc., USA) and phenylephrine 5% (Bausch & Lomb Pharmaceuticals Inc., USA).

Intravitreal injection and surgical technique. Two surgical techniques were used to make the subretinal or intravitreal injection application of the dye. In a first technique, the subretinal toxicity of the dye was evaluated. Anesthesia was performed by intramuscular injection of 35 mg / kg of xylazine hydrochloride and 5 mg / kg of ketamine hydrochloride, followed by placement of a blepharostat in the right eye of the animals and a drop of topical povidone-iodine 5% for preparation of the eye for vitrectomy with 3 sclerotomies. An infusion was positioned at the temporal sclera to maintain the intraocular pressure at 30 mmHg and fixed by Vycril 7-0 sutures (Ethicon, Inc.). Then two nasal and superior temporal sclerotomies 2.5 mm posterior to the limbus were performed. Pars plana vitrectomy was performed using a 20-gauge system with the vitrectiomia AccurusTM - (Alcon, Fort Worth, Texas, USA) instrument.

The next surgical step was the application of two marks of diode laser photocoagulation FTC / TTT / IPDT 2000TM (Electronica Opto, São Carlos, SP, Brazil) with the parameters of 100 µm in diameter, 100 milliwatts and duration of 300 milliseconds, in the temporal and nasal retina 4 mm below the optic nerve. The function of the laser marks is to delimit the region that the dye is injected into the subretinal for histological analysis. The administration of the dye was made with a polyamide 41-gauge infusion cannula for macular translocation (Bausch & Lomb, USA). The cannula was positioned 2 mm above the prior laser photocoagulation mark. The dye to be used was injected into the subretinal space at different concentrations, and at 2 mm below the mark of temporal photocoagulation. The dye was infused continuously until a bubble of 3 mm in diameter is formed by an infusion of 0.02 mL of the dye. Next, 0.02 mL of BSS control solution was infused with the same shape than the nasal laser mark. The eye was sutured with 7-0 Vicryl thread in the three sclerotomies. This experiment was repeated in two animals with each dye at different concentrations. The rabbits were sacrificed with intravenous injection of 2 mL of phenobarbital and the eyes were removed by laser enucleation 24 hours after surgery.

In a second experiment, the toxicity of intravitreal dye was evaluated. A total of 0.1 mL of dye was injected into the vitreous cavity of eyes and repeated in two additional animals. In the contralateral eye of an animal was injected 0.1 mL of BSS (290 mOsm) as control. The technique of intravitreal injection follows international patterns²². Briefly, a blepharostat was placed followed by instillation of a drop of 5% povidone-iodine on the eye. Under the surgical microscope, a 27-gauge needle was connected to a 1 mL syringe containing the dye or BSS and was injected in the superotemporal sclera region, 2 mm posterior to the limbus. The eyes were examined to exclude retinal detachment, vitreous hemorrhage, or injury to the lens. At the end of the procedure, a drop of sterile eye drops with antibiotics and steroids were applied. The rabbits were sacrificed with intravenous injection of 2 mL of phenobarbital and the eyes were removed by laser enucleation 24 hours after the injection and fixed in glutamaldehyde at 2.5%.

Fundoscopy and Fluorescein Angiography. Fluorescein angiography was performed by intravenous injection for 30 seconds of 0.3 mL of sodium fluorescein 10% in the auricular vein of the animal magna. Angiography was performed using a specific camera eye fundus (Topcon TRC, Topcon, Tokyo, Japan) every 20 seconds up until 5 minutes after initial injection. An examination of fluorescein angiography was performed 6 hours and 24 hours after intravitreal application of dye. The dye injection was performed slowly to avoid iatrogenically breaking the hematorretinian barrier by rapid bolus injection that can happen in these animals.

Histology. The eyes were enucleated and fixed in a solution of 2% paraformaldehyde, 2% glutaraldehyde and phosphate buffer 0.1 mol/L and pH 7.4 for 24 hours. This was followed by soaking the tissue in Araldite® for histological sections. The material was collected from two different areas at sites of previous injections of the dye. All samples were collected in areas separated 500 µm from each other. The samples were analyzed by optical microscopy and electron microscopy. For optical microscopy, the thin tissue sections were examined after staining with toluidine blue dye. For electron microscopy ultrathin sections were placed in copper microstructures, stained with uranyl acetate and silver citrate, and examined with electron microscope Jeol JX 1500TM (Jeol Ltd., Tokyo, Japan).

The following describes the actions taken at each point of the study:
Time zero: Antisepsis and anesthesia of the animal, intravitreal injection / subretinal dye (experimental group), and intravitreal injection / subretinal balanced salt solution (control group).
Time 6 hours: Fundus fluorescein angiography and retinal examination.
Time 24 hours: Fundus fluorescein angiography and retinal examination, asepsis and anesthesia of the animal; sacrifice the animal by an overdose of pentobarbital.

After the procedure: Data collection for histology, electron microscopy and statistical analysis of results.

Time 7 days: Fundus fluorescein angiography and retinal examination, asepsis and anesthesia of the animal, and sacrifice the animal by an overdose of pentobarbital.

### Example 7 - Use as Histopathological Stain.

The present compositions are useful as dyes for histopathological staining, both in vitro and on the external surface of the eye. Dye compositions used were a dispersion of lutein and zeaxanthin (L/Z) 0.1-0.7% associated with brilliant blue 0.0125%-0.05% in a aqueous buffer solution at pH range [6-7.4] and osmolality range of [280-320] Osm/Kg. 2ml of these solutions were packaged in 3ml-vials. Tissues used for staining were conjunctiva, cornea, lids and retinal epithelial tissue. The staining was done by the following process: the whole eye globe was obtained and immersed in a Karnovski fixative composed with glutaraldehyde 2.5% and paraformaldehyde 2% in sodium cacodylate 0.1M buffer keeping the solution at pH 7.2.

The solution was maintained for 24hours and retinal tissue, conjunctiva and cornea tissues were cut. Then, the first rinse process was conducted three times by using a sodium cacodylate 0.1M buffer during 15 minutes. The second rinse process was then conducted leaving the tissue in solution overnight with the same buffer. The second fixative process is conducted with osmium tetroxide 2% in sodium cacodylate 0.1M buffer for 2h, followed by a 10 minute rinse with same buffer. After that, the material went through the dehydration/drying process, starting with ethanol 70% (15min, twice), 90% (15min, twice) and 100% (15min, 4 times) and finishing with propylene oxide (30min, 3 times). The material was then submitted to inflitration with Epon® resin (Miller-Stephenson) in a 1:1 solution of Epon®:propylene oxyde overnight, with lid open. The day after, the material was re-infiltered with Epon® for 4h in vacuum conditions. After this, the material was included in a mold and taken to the polimerization process at 60 °C for 48h. After trimming, semi-thin cuts were made with 40 micrometer thickness in a Leyca Reichert Ultracut #702501 microtome and finally stained with the dye solutions mentioned above. There was an adequate staining of the material cut providing a greenish blue staining to all materials.

### Example 8 - Protection against tissue damage during surgical procedures.

Surgical procedures normally involve an exposure of tissues and cells to the surgical room ambient, which typically has oxygen and light. Specifically, most surgical rooms possess powerful illuminators (endoilluminators) whose only function is to provide the best illumination possible for the surgeon to visualize the target areas and be able to perform with no problem all surgical steps. The endoilluminator is described in scientific literature as a powerful source of blue light which can provide phototoxic effect in the retina.³² Blue light is described in scientific literature as an inherent source of free radicals.³³ Free radicals are extensively described in the literature as attackers of the integrity of photoreceptor lipophilic membrane, causing premature cell death, and leading to age-related macular degeneration, the leading cause of blindness in the western world. Free radicals also provide for the accumulation of drusen in the lens leading to premature onset of cataracts.³⁴ Lutein and Zeaxanthin (L/Z) can stabilize, quench or scavenge free radicals avoiding further damages to phtotoreceptor or lens integrity and therefore contributing to decrease the severity or even incidence of these conditions. On the other hand L/Z have the property to absorb blue wavelengths of light in the area of the 446 nm therefore diminishing the exposure of lens or retinal photoreceptors to the hazardous light (3-23). The next paragraph portrays a description of L/Z mechanism of action. The endoilluminator is a source of intense light exposure, mainly blue light. The surgical room is full of oxygen, the perfect fuel for free radical formation. The intraocular use of L/Z solutions will provide an extra protection to the surgery given by its filter and antioxidant mechanism of action. Costa et al conclude that "Light-induced retinal toxicity by the endoilluminator is dependent on factors such as the duration of use, type, power, and wavelength of light source. The hazard of light radiation increases with decreasing wavelength because stronger phototoxic effects are mediated by ultraviolet rays and blue light, as shown in experimental studies." Moreover, since oxidation is a systemic problem that can happen anywhere in human body tissues and since exposure to the endoilluminator is present in all surgeries, not only in ophthalmic surgeries, the presence of L/Z as a surgical aid can diminish the contact and attack of blue light photons and free radicals to the tissues, accelerating its degenerations (and consequent degenerative diseases), in virtually all parts of the body.

Lutein and zeaxanthin are lipophilic pigments belonging to the group of carotenoids and traditionally found in fruits and vegetables. These two carotenoids are structural isomers and have a hydroxyl group in the terminal portion of the molecule, which partly explains their different polarity and tropism for certain biological structures, such as the macula. There is a significant array of published peer-reviewed prior studies showing that L/Z are associated with the possible prevention of age-related maculopathies due (i) to its antioxidant mechanism, which can prevent progress of macular degeneration and (ii) to its exclusive distribution in the macula. The selective and specific distribution of these carotenoids suggests that they play an important role in this region, both as a "filter" of blue light and as an antioxidant. Blue light is well-known to be one of the major generators of hydroxyl radical, a reactive oxygen specimen that will attack biological structures such as photoreceptors. Reactive Oxygen Species (ROS) are continuously generated in the retina by the existence of several metabolic reactions, considerable oxygen levels, polyunsaturated fatty acids, and photons of blue light. The continuous exposure to these photons in the retina will lead to the formation of ROS which, in its quest for stabilization, will capture an electron of any photosensitizer such as a photoreceptor, attacking this structure. With an extensive polyenic chain, L/Z, if present in the lens and retinal region, can donate the electron needed to the ROS formed as a result of the blue light presence, quenching the triplet stage of both photosensitizers as well as singlet oxygen, inhibiting further formation of ROS and preventing lipo-peroxydation. Interestingly, L/Z molecules absorb light in the region of the visible spectrum, around 446nm, which coincides exactly with the blue region of the electromagnetic spectrum. For this reason, it is known that L/Z may be natural absorbers of blue light, diminishing its negative prevalence in the lens and retina. The use of oral supplements of L/Z with the objective of preventing age-related maculopathies has been registered for several years.

### Example 10 - Dye formulation including L/Z and Brilliant Blue (BB)/Trypan Blue (TB)

Referring to Fig. 1, based on the principle of the colorimeter, the *a* axis is from green to red, the *b* axis is from blue to yellow and the *L* axis is from black to white. L/Z has an orange color, so its color is between yellow (b+) and red (a+); BB and TB each have a blue color, so its resulting color falls between blue (b-) and green (a-). In order to obtain a green color for the solution, there is a need to increase the amount of BB / TB or decrease the amount of L/Z to obtain a negative *a* value. But in order to decrease toxicity of formulation, it is necessary to increase the amount of L/Z and decrease the amount of BB / TB.

Based on the experiments, a combination in the ratio L/Z 0.3%+BB0.025% (close to 10:1) has a good green color and possess high dying activity and low toxicity. L/Z 0.3%+TB 0.1% (3:1) also has an interesting green color and intense dying activity but some toxicity. In order to decrease the amount of TB to 0.05%, we must accept a little dark green color solution (L/Z 0.5%+TB 0.05%) or accept a little low dying activity (L/Z 0.15%+TB0.05%). A 10:1 L/Z-BB ratio is optimal for color/toxicity. A 3:1 L/Z - TB ratio is optimal for color but not for toxicity. The 10:1 ratio seems the best balance between color/solubilization/toxicity with either solution. Also, concentrations of BB below 0.025% and of TB below 0.05% seem to have staining efficacy that was impaired in cadaveric eyes. Additionally, concentrations of L/Z above 0.5% can result in precipitation from the solution and pose a challenge to dissolution. Furthermore, it appears that the L/Z limiting concentration is 0.5% in each combination either with BB or TB using different concentrations of buffer, stirring time and temperature.

### References

1. Heij ECL, Hendrikse F, Kessels AGH, Derhaag PJFM: Vitrectomy results in diabetic macular edema without evident vitreomacular traction. Graefes Arch Clin Exp Ophthalmol 2001;239:264-70.
2. Gandorfer A, Messmer EM, Ulbig MW, Kampik A: Resolution of diabetic macular edema after surgical removal of the posterior hyaloid and the inner limiting membrane. Retina 2000;20:126-133.
3. Rodrigues EB, Meyer CH, Kroll P. Chromovitrectomy: a new field in vitreoretinal surgery. Graefes Arch Clin Exp Ophthalmol 2005;243:291-3.
4. Burk SE, Da Mata AP, Snyder ME, et al. Indocyanine green-assisted peeling of the retinal internal limiting membrane. Ophthalmology 2000;107:2010-2014.
5. Gandorfer A, Messemer EM, Ulbig MW, et al: Indocyanine green selectively stains the internal limiting membrane. Am J Ophthalmol 2001;131:387-88.
6. Rodrigues EB, Meyer CH, Farah ME, Kroll P. Intravitreal staining of the internal limiting membrane using indocyanine green in the treatment of macular holes. Ophthalmologica 2005;219:251-62.
7. Enaida H, Sakamoto T, Hisatomi T, et al: Morphological and functional damage of the retina caused by intravitreous indocyanine green in rat eyes. Graefes Arch Clin Exp Ophthalmol 2002;240:209-13.
8. Maia M, Margalit E, Tso MOM, et al: Effects of intravitreal indocyanine green injection in rabbits. Retina 2004;24:69-79.
9. Maia M, Kellner L, de Juan E, Jr., et al: Effects of indocyanine green injection on the retinal surface and into the subretinal space in rabbits. Retina 2004;24:80-91.
10. Stalmans P, Van Aken EH, Melles G, et al: Trypan blue not toxic for retinal pigment epithelium in vitro. Am J Ophthalmol 2003;135:234-36.
11. Rodrigues EB, Costa EF, Penha FM, Melo GB, Bottós J, Dib E, Furlani B, Lima VC, Maia M, Meyer CH, Höfling-Lima AL, Farah ME. SurvOphthalmol. 2009 Sep-Oct;54(5):576-617. Dyes in ocular surgery: principles for use in chromovitrectomy.
12. Farah ME, Maia M, Rodrigues EB. Am J Ophthalmol. 2009 Sep;148(3):332-40. Epub 2009 May 24.
13. Rodrigues EB, Penha FM, Farah ME, de Paula Fiod Costa E, Maia M, Dib E, Bottós J, Freymuller E, Furlani B, Meyer CH, Magalhães O Jr, Lima-Filho AA, Safatle A. Retina. 2009 Apr;29(4):497-510.
14. Costa Ede P, Rodrigues EB, Farah ME, Dib E, Penha F, Magalhães O Jr, Furlani BA, Lima Filho AA, de Miranda A, Maia M. Vital dyes and light sources for chromovitrectomy: comparative assessment of osmolarity, pH, and spectrophotometry. Current concepts of trypan blue in chromovitrectomy.Invest Ophthalmol Vis Sci. 2009 Jan;50(1):385-91. Epub 2008 Aug 8.
15. Maia M, Penha FM, Farah ME, Dib E, Príncipe A, Lima Filho AA, Magalhães O Jr, Freymüller E, Rodrigues EB. Subretinal injection of preservative-free triamcinoloneacetonide and supernatant vehicle in rabbits: an electron microscopy study.Graefes Arch Clin Exp Ophthalmol. 2008 Mar;246(3):379-88. Epub 2007 Dec 11.
16. Maia M, Penha F, Rodrigues EB, Príncipe A, Dib E, Meyer CH, Freymuller E, Moraes N, Farah ME. Curr Eye Res. 2007 Apr;32(4):309-17.
17. Maia M, Farah ME, Belfort RN, Penha FM, Lima Filho AA, Aggio FB, Belfort R Jr. Effects of intravitreal triamcinolone acetonide injection with and without preservative. Effects of subretinal injections of indocyanine green, trypan blue, and glucose in rabbit eyes.Br J Ophthalmol. 2007 Sep;91(9):1122-4. Epub 2007 Mar 23.
18. Penha FM, Maia M, Eid Farah M, Príncipe AH, Freymüller EH, Maia A, Magalhães O Jr, Smith RL Ophthalmology. 2007 May;114(5):899-908. Epub 2007 Feb 8.
19. Farah, M. E., Maia, M, Rodrigues, E. B. Dyes in Ocular Surgery: Principles for Use in Chromovitrectomy. American Journal of Ophthalmology, v.23, p.1 - 1, 2009. Referências adicionais: Inglês. Meio de divulgação: Impresso, Home page: doi:10.1016/j.ajo.2009.04.003].
20. Rodrigues, E. B., Penha, F. M., Farah M. E., Costa, E. F., Maia, M, dib, E., Bottos, J. M., Freymuller, E., Furlani, B. A., Meyer, C., Magalhães Jr., O., Lima-Filho, A. A. S., Safatle, A. Preclinical investigation of the retinal biocompatibility of six novel vital dyes for chromovitrectomy. Retina (Philadelphia), v.29, p.497 - 510, 2009. Referências adicionais: Inglês. Meio de divulgação: Impresso.
21. Maia, M, Farah M. E., Rodrigues, E. B., Malerbi, F. Subretinal brilliant blue G migration during internal limiting membrane peeling. British Journal of Ophthalmology, v.93, p.330 - 331, 2009. Referências adicionais: Inglês. Meio de divulgação: Impresso.
22. Dib, E., Rodrigues, E. B., Maia, M, Meyer, C., Penha, F. M., Furlani, B. A., Farah, M. E. Vital Dyes in chromovitrectomy. Arquivos Brasileiros de Oftalmologia (Impresso), v.72, p.845 - 850, 2009. Referências adicionais: Inglês. Meio de divulgação: Impresso.
23. Penha, F. M., Maia, M., Farah, M. E., Dib, E., Príncipe, A. H., Devin, F., Rodrigues, E. B., Duprat, J. P., Freymüller, E. Morphologic and Clinical Effects of Subretinal Injection of Indocyanine Green and Infracyanine Green in Rabbits. Journal of Ocular Pharmacology and Therapeutics, v.24, p.52 - 61, 2008. Referências adicionais: Inglês. Meio de divulgação: Meio digital (DOC. F2).
24. Maia, M., Penha, F. M., Farah, M. E., Dib, E., Principe, A., Lima Filho, A. S., Magalhaes Jr., O., Freymuller, E., Rodrigues, E. B. Subretinal injection of preservative-free triamcinolone acetonide and supernatant vehicle in rabbits: an electron microscopy study. Graefe's Archive for Clinical and Experimental Ophthalmology, v.246, p.379 - 388, 2008. Referências adicionais: Inglês. Meio de divulgação: Meio digital (DOC. F4).
25. Rodrigues, E. B., Maia, M., Penha, F. M., Dib, E., Bordon, A. F., Magalhães JR., O., Farah, M. E. Técnica para injeção intravítrea de drogas no tratamento de doenças vítreorretinianas. Arquivos Brasileiros de Oftalmologia, v.71, p.902 - 907, 2008. Referências adicionais: Inglês. Meio de divulgação: Meio digital, Home page: doi:10.1590/s0004-27492008000600028].
26. Rodrigues, E. B., Maia, M., Penha, F. M., Dib, E., Bordon, A. F., Magalhães JR., O., Farah, M. E. Técnica para injeção intravítrea de drogas no tratamento de doenças vítreorretinianas. Arquivos Brasileiros de Oftalmologia, v.71, p.902, 2008. Referências adicionais: Português. Meio de divulgação: Meio digital, Home page:[doi:10.1590/s0004-27492008000600028].
27. Penha, F. M., Maia, M., Farah, M. E., Kawakami, L. T., Hofling-Lima, A. L. Thermal damage to a light probe using a xenon light source. Acta Ophthalmologica Scandinavica, v.86, p.461 - 462, 2008. Referências adicionais: Inglês. Meio de divulgação: Meio digital (DOC. F5).
28. Massanat, Y., Shenoy, H., Speirs, V., Hanby, A., Horgan, K. Properties and characteristics of the dyes injected to assist axillary sentinel node localization in breast surgery. European Journal of Surgical Oncology, 2006, vol. 32, no4, pp. 381-384.
29. Prien, S.D., Dunn, C., Messer, R. H. Adhesion-promoting properties of dyes routinely used during fertility surgeries. Journal of Assisted Reproduction and Genetics, 1995, Volume 12, Number 2, 136-140.
30. Kim, S. H., Milson, J.M. Perioperative tumor localization for laparoscopic colorectal surgery Surgical Endoscopy, 1997, Volume 11, Number 10, 1013-1016.
31. Sorsby, A., Wright, A. D., Vital Staining in Brain Surgery. A Preliminary Note Proc R Soc Med. 1943 January; 36(3): 137-140.
32. Costa E.dP.F, Rodrigues E.B., Farah M.E., Dib E., Penha F., Magalhaes, O. Jr, et al. Vital Dyes and Light Sources for Chromovitrectomy: Comparative Assessment of Osmolarity, pH, and Spectrophotometry. IOVS. 2009;50:385-91.
33. Beatty S., Boulton M., Henson D., Koh H.H., and Murray I.J. Macular pigment and age related macular degeneration. Br J Ophthalmol 83: 867-877, 1999. Antioxidant status and neovascular age-related macular degeneration. Eye Disease Case-Control Study Group. Arch Ophthalmol 111: 104-109, 1993.
34. Brown L., Rimm E.B., Seddon J.M., Giovannucci E.L., Chasan-Taber L., Spiegelman D., Willett W.C., and Hankinson S.E. A prospective study of carotenoid intake and risk of cataract extraction in US men. Am J Clin Nutr 70: 517-524, 1999.
35. Acosta M.M., Boyce H.W. Chromoendoscopy-where is it useful? J Clin Gastroenterol. 1998;27:13-20.
36. Sorsby A. Vital staining of the fundus. Trans Ophthal Soc UK. 1939;59:727-730.
37. Gifford H. Use of fluorescein intravenously as an aid to ophthalmic diagnosis and treatment. Arch Ophthalmol. 1940;24:122-131.
38. Rodrigues, E, and Meyer, C. Current Dyes in Chromovitrectomy: Pros and Cons May 2005 http://www.retinalphysician.com/article.aspx?article=100086.

## Claims

1. A natural dye selected from the group consisting of lutein, zeaxanthin, urucum, lycopene, astaxanthin, beta-carotene, and beta-cryptoxanthin, alone or in combination with each other for use in staining ocular membranes or structures in intraocular surgery.

2. A dye for use as defined in claim 1, further comprising a second dye selected from the group consisting of brilliant blue, indocyanine green, infracyanine green, trypan blue, patent blue, bromophenol blue, fast green, indigo carmine, evans blue, light green, triamcinolone, crystal violet, fluorescein, fluormetolone acetate and congo red and combinations thereof.

3. A dye for use as defined in claim 1, wherein said dye consists of lutein and zeaxanthin.

4. A dye for use as defined in claim 1, wherein the ocular membranes or structures comprise the anterior and posterior capsule, corneal epithelium and endothelium, epiretinal membrane, internal limiting membrane, vitreous or posterior hyaloid.

5. A composition for use in staining ocular membranes or structures in intraocular surgery comprising a natural dye selected from the group consisting of lutein, zeaxanthin, urucum, lycopene, astaxanthin, beta-carotene, and beta-cryptoxanthin, alone or in combination with each other and a pharmaceutically acceptable vehicle.

6. A composition for use according to claim 5, further comprising a dye selected from the group consisting of brilliant blue, indocyanine green, infracyanine green, trypan blue, patent blue, bromophenol blue, fast green, indigo carmine, evans blue, light green, triamcinolone, crystal violet, fluorescein, fluormetolone acetate and congo red and combinations thereof.

7. A composition for use according to claim 6, wherein the dye is present in a concentration of 0.001% to 20%.

8. A composition for use according to claim 5, wherein the natural dye consists of lutein and zeaxanthin, alone or combined.

9. A composition for use according to claim 5, wherein the vehicle is selected from the group consisting of phosphate buffer, polyvinyl alcohol, benzyl alcohol, purified water, sodium phosphate, sodium chloride , calcium chloride, magnesium chloride, potassium chloride, sodium citrate, sodium acetate, boric acid, sodium borate, methyl cellulose, hyaluronic acid, dextran, sodium polysorbate, chondroitin sulfate, sodium edetate, propylene glycol, polyethylene glycol, phenyl sodium, potassium phthalate, potassium phosphate, citric acid, , metaphosphoric acid, glycocholic acid, acetic acid, and combinations thereof.

10. A composition comprising lutein and zeaxanthin for intraocular use in protecting tissues and cells exposed during surgical procedures from damage due to light.

## Patentansprüche

1. Natürlicher Farbstoff ausgewählt aus der Gruppe bestehend aus Lutein, Zeaxanthin, Urucum, Lycopin, Astaxanthin, Betacarotin und Beta-Cryptoxanthin, allein oder in Kombination miteinander zur Verwendung beim Färben von okularen Membranen oder Strukturen in intraokularer Chirurgie.

2. Farbstoff zur Verwendung gemäß Definition in Anspruch 1, ferner umfassend einen zweiten Farbstoff ausgewählt aus der Gruppe bestehend aus Brillantblau, Indocyaningrün, Infracyaningrün, Trypanblau, Patentblau, Bromphenolblau, Echtgrün, Indigokarmin, Evans-Blau, Hellgrün, Triamcinolon, Kristallviolett, Fluoreszein, Fluormetolonacetat und Kongorot und Kombinationen davon.

3. Farbstoff zur Verwendung gemäß Definition in Anspruch 1, wobei der Farbstoff aus Lutein und Zeaxanthin besteht.

4. Farbstoff zur Verwendung gemäß Definition in Anspruch 1, wobei die okularen Membranen oder Strukturen die vordere und hintere Kapsel, das Hornhautepithel und Hornhautendothel, die epiretinal Membran, intern begrenzende Membran, den Glaskörper oder hinteren Hyaloid umfasst.

5. Zusammensetzung zur Verwendung beim Färben von okularen Membranen oder Strukturen in intraokularer Chirurgie, umfassend einen natürlichen Farbstoff ausgewählt aus der Gruppe bestehend aus Lutein, Zeaxanthin, Urucum, Lycopin, Astaxanthin, Betacarotin und Beta-Cryptoxanthin, allein oder in Kombination miteinander und ein pharmazeutisch annehmbares Vehikel.

6. Zusammensetzung zur Verwendung nach Anspruch 5, ferner umfassend einen Farbstoff, ausgewählt aus der Gruppe bestehend aus Brillantblau, Indocyaningrün, Infracyaningrün, Trypanblau, Patentblau, Bromphenolblau, Echtgrün, Indigokarmin, Evans-Blau, Hellgrün, Triamcinolon, Kristallviolett, Fluoreszein, Fluormetolonacetat und Kongorot und Kombinationen davon.

7. Zusammensetzung zur Verwendung nach Anspruch 6, wobei der Farbstoff in einer Konzentration von 0,001 % bis 20 % vorhanden ist.

8. Zusammensetzung zur Verwendung nach Anspruch 5, wobei der natürliche Farbstoff aus Lutein und Zeaxanthin, allein oder kombiniert, besteht.

9. Zusammensetzung zur Verwendung nach Anspruch 5, wobei das Vehikel ausgewählt ist aus der Gruppe bestehend aus Phosphatpuffer, Polyvinylalkohol, Benzylalkohol, aufgereinigtem Wasser, Natriumphosphat, Natriumchlorid, Calciumchlorid, Magnesiumchlorid, Kaliumchlorid, Natriumzitrat, Natriumacetat, Borsäure, Natriumborat, Methylcellulose, Hyaluronsäure, Dextran, Natriumpolysorbat, Chondroitinsulfat, Natriumedetat, Propylenglycol, Polyethylenglycol, Phenylnatrium, Kaliumphthalat, Kaliumphosphat, Zitronensäure, metaphosphorsäure, Glycocholsäure, Essigsäure und Kombinationen davon.

10. Zusammensetzung, umfassend Lutein und Zeaxanthin zur intraokularen Verwendung beim Schutz von Geweben und Zellen, die während chirurgischer Verfahren einer Beschädigung durch Licht ausgesetzt sind.

## Revendications

1. Colorant naturel sélectionné dans le groupe comprenant : lutéine, zéaxanthine, urucum, lycopène, astaxanthine, bêta-carotène et bêta-cryptoxanthine, seul ou en combinaison, pour utilisation dans la coloration des membranes ou structures oculaires en chirurgie intraoculaire.

2. Colorant pour utilisation comme définie dans la revendication 1, comprenant en outre un deuxième colorant sélectionné dans le groupe comprenant : bleu brillant, vert d'indocyanine, vert d'infracyanine, bleu trypan, bleu patente, bleu de bromophénol, vert rapide, carmin d'indigo, bleu d'evans, vert clair, triamcinolone, violet cristal, fluorescéine, acétate de fluormétolone et rouge Congo et des combinaisons de ceux-ci.

3. Colorant pour utilisation comme définie dans la revendication 1, ledit colorant étant constitué de lutéine et de zéaxanthine.

4. Colorant pour utilisation comme définie dans la revendication 1, les membranes et structures oculaires comprenant la capsule antérieure et postérieure, l'épithélium et l'endothélium cornéens, la membrane épirétinienne, la membrane limitante interne, la membrane hyaloïde vitrée ou postérieure.

5. Composition pour utilisation dans la coloration des membranes ou structures oculaires en chirurgie intraoculaire comprenant un colorant naturel sélectionné dans le groupe comprenant : lutéine, zéaxanthine, urucum, lycopène, astaxanthine, bêta-carotène, et bêta-cryptoxanthine, seul ou en combinaison, et un excipient de qualité pharmaceutique.

6. Composition pour utilisation selon la revendication 5, comprenant en outre un colorant sélectionné dans le groupe comprenant : bleu brillant, vert d'indocyanine, vert d'infracyanine, bleu trypan, bleu patente, bleu de bromophénol, vert rapide, carmin d'indigo, bleu d'evans, vert clair, triamcinolone, violet cristal, fluorescéine, acétate de fluormétolone et rouge Congo et des combinaisons de ceux-ci.

7. Composition pour utilisation selon la revendication 6, dans laquelle le colorant est présent en concentration de 0,001 % à 20 %.

8. Composition pour utilisation selon la revendication 5, dans laquelle le colorant naturel est constitué de lutéine et de zéaxanthine, seule ou combinée.

9. Composition pour utilisation selon la revendication 5, dans laquelle l'excipient est sélectionné dans le groupe comprenant : phosphate tampon, poly(alcool de vinyle), alcool benzylique, eau purifiée, phosphate de sodium, chlorure de sodium, chlorure de calcium, chlorure de magnésium, chlorure de potassium, citrate de sodium, acétate de sodium, acide borique, borate de sodium, méthylcellulose, acide hyaluronique, dextrane, polysorbate de sodium, sulfate de chondroïtine, EDTA sodique, propylène glycol, polyéthylène glycol, phényle-sodium, phtalate de potassium, phosphate de potassium, acide citrique, acide métaphosphorique, acide glycocholique, acide acétique et des combinaisons de ceux-ci.

10. Composition comprenant de la lutéine et de la zéaxanthine pour utilisation intraoculaire dans la protection contre les dommages dus à la lumière des tissus et cellules exposés pendant les interventions chirurgicales.
